# EUROPÄISCHE PATENTSCHRIFT

(11) **EP 0 687 164 B1**
(45) Veröffentlichungstag und Bekanntmachung des Hinweises auf die Patenterteilung: **07.05.1997**
(21) Anmeldenummer: 95903295.4
(22) Anmeldetag: 28.11.1994
(51) Int. Cl.: A61F 2/06

(54) **GEFÄSSPROTHESE**
VASCULAR PROSTHESIS
PROTHESE VASCULAIRE

(30) Priorität: 30.11.1993 DE 4340755
(43) Veröffentlichungstag der Anmeldung: 20.12.1995
(73) Patentinhaber: Zurbrügg, Heinz Robert, Dr. med., CH-3047 Bremgarten (CH)
(72) Erfinder: Zurbrügg, Heinz Robert, Dr. med., CH-3047 Bremgarten (CH)
(74) Vertreter: Füchsle, Klaus, Dipl.-Ing.
(86) Internationale Anmeldenummer: EP9403935
(87) Internationale Veröffentlichungsnummer: WO9515130

(56) Entgegenhaltungen:
- EP-A- 0 492 481
- DE-B- 1 766 921
- FR-A- 2 556 210
- US-A- 3 337 673

## Beschreibung

Die Erfindung betrifft eine Gefäßprothese für den Ersatz von Blutgefäßen, insbesondere arterielle Blutgefäße, im menschlichen oder auch tierischen Körper, welche aus einem von einem menschlichen oder tierischen Körper entnommenen Abschnitt eines Blutgefäßes, z.B. einer Vene, und einem über diesen Gefäßabschnitt gezogenen Netzschlauch besteht. Gefäßprothesen dieser Gattung sind bereits bekannt (z.B. EP-0 055 250 B1 und FR-A-25 56 210).

Der Oberbegriff des Anspruchs 1 geht aus von einer Gefäßprothese gemäß FR-A-2 556 210.

Bei diesen Prothesen dient nach Implantation der Netzschlauch dazu, den Gefäßabschnitt an seiner Außenseite abzustützen und damit ein unerwünschtes Aufweiten durch Blutdruck Zu vermeiden. Die Erfindung betrifft ferner ein Verfahren sowie ein Teile-Set zur Herstellung einer solchen Gefäßprothese.

Die bekannten Gefäßprothesen der eingangs genannten Art haben Netzschläuche, die aus miteinander verwebten Fäden bestehen, wobei sie entweder einen weitgehend unveränderbaren Durchmesser haben oder mit gleichmäßigen, also unveränderlichem Durchmesser auf dem Ersatzgefäß sitzen. Da die Ersatz-Blutgefäße einen sich über ihre Länge verändernden, ungleichmäßigen Durchmesser haben, haben die bekannten Netzschläuche den Nachteil, daß sie sich nicht oder nur mangelhaft an diese unterschiedlichen Gefäßdurchmesser anpassen können, so daß sie stellenweise nicht an der Außenwand des Ersatzgefäßes zum Anliegen gelangen und damit an diesen Stellen nicht die erforderliche Stützfunktion für das Ersatzgefäß erfüllen können. Nach Implantation der Prothese in den menschlichen Körper kommt es daher an diesen Stellen zu einer unerwünschten Aufweitung des Ersatzgefäßes und einer damit verbundenen Dehnung unter gleichzeitiger Verdünnung der Gefäßwand oder es tritt eine Vernarbung an diesen Schwachstellen auf, die das Gefäßvolumen einengt.

Andererseits besteht auch wegen der unveränderbaren Durchmesser der bekannten Netzschläuche die Gefahr, daß der Netzschlauch das in ihm befindliche Ersatzgefäß an dickeren Wandstellen oder an Seitenästen des Gefäßes eingeengt, wodurch der Innendurchmesser der Prothese verringert wird, was aus medizinischer Sicht höchst unerwünscht ist. Außerdem ist bei bogenförmiger Implantation der Prothese die Gefahr des Einknickens der Prothese gegeben, was ebenfalls aus medizinischer Sicht zu vermeiden ist.

Der Erfindung liegt daher die Aufgabe zugrunde, diesen Nachteil bei einer Gefäßprothese der eingangs genannten Gattung zu vermeiden, d.h. eine Gefäßprothese aus einem mit einem Netzschlauch überzogenen Ersatzgefäßabschnitt zu schaffen, bei der das Ersatzgefäß über seine gesamte Länge durch den Netzschlauch vollflächig abgestützt ist, bei welcher sich also der Netzschlauch über die gesamte Länge und den gesamten Umfang des Ersatzgefäßes im wesentlichen gleichmäßig an die unregelmäßig geformte Gefäßaußenwand stützend anlegt, so daß eine stellenweise radiale Dehnung der Ersatzgefäßwand durch den Druck des durch die Prothese hindurchströmenden Blutes oder ein radiales Nachinnen-Drücken der Gefäßwand durch den übergezogenen Netzschlauch unter Verringerung des Innendurchmessers der Prothese im wesentlichen ausgeschlossen ist.

Diese Aufgabe wird bei einer Gefäßprothese der eingangs genannten Gattung gemäß Anspruch 1 dadurch gelöst, daß der Netzschlauch auf dem Ersatzgefäß stellenweise in Längsrichtung unter Durchmesserveränderung gedehnt oder gestaucht und dadurch zum ganzflächigen, gleichmäßigen Anliegen an dem Ersatzgefäß gebracht ist. Zu diesem Zweck ist der Netzschlauch so beschaffen, daß sein Durchmesser durch axiales Dehnen bis auf das Zehnfache des Durchmessers des entspannten Schlauches verkleinert und durch axiales Stauchen bis auf das Fünffache des Durchmessers des entspannten Schlauches vergrößert werden kann.

Durch das Dehnen oder Stauchen des Netzschlauches in Längsrichtung wird der Durchmesser des Netzschlauches unter gegenseitiger Lageänderung seiner im Verbund zueinander verschiebbaren Netzfäden stellenweise, d.h. an bestimmten Längsstellen, in seinem Durchmesser aufgeweitet oder verengt, wodurch er an der gesamten Oberfläche des Ersatzgefäßes gleichmäßig zum Anliegen gebracht werden kann. Durch dieses ganzflächige, gleichmäßige, satte Anliegen des Netzschlauches an dem Ersatzgefäß wird eine unterschiedliche Dehnung der Wand des Ersatzgefäßes durch den Druck des in den Kreislauf des Patienten eingesetzten Prothese strömenden Blutes verhindert, da der Netzschlauch den Gefäßabschnitt an allen Stellen gleichmäßig abstützt. Außerdem wird aus dem gleichen Grunde bei bogenförmiger Implantation die Gefahr der Knickung der Prothese weitgehend ausgeschlossen. Auch wird an denjenigen Stellen, an denen die Gefäßwand besonders dick ist oder Seitenäste besitzt, ein radiales Zusammendrücken der Gefäßwand unter Verringerung des Innendurchmessers der Prothese verhindert.

Die Netzfäden können eine Fadenstärke von 10 bis 200 µm haben und aus Metall oder einer Metallegierung, vorzugsweise aus rostfreiem Stahl, aber auch aus biokompatiblem Kunststoff bestehen. Sie können auch eine Beschichtung aus biokompatiblem Material, z.B. Metall oder Metallegierung, haben. Vorzugsweise haben die Netzfäden kreisförmigen Querschnitt. Der Querschnitt kann aber auch elliptisch, dreieckig oder mehreckig, trapez- oder trapezoidförmig, rhombus- oder rhomboidförmig sein.

Vorteilhaft kann der Netzschlauch 11 bis 201 Fäden haben, wobei jeder Faden individuelle Merkmale aufweisen kann. Vorzugsweise sind die Netzfäden des Netzschlauches in gewissem Ausmaß gegeneinander verschiebbar. Die Oberfläche der Fäden sollte möglichst glatt sein, d.h. in tausendfacher Vergrößerung mit dem Rasterelektronenmikroskop keine Poren erkennen lassen, die größer als 0,5 µm sind. Die Oberfläche der Metallfäden kann elektropoliert sein.

Der Durchmesser der Netzfäden sollte über ihre gesamte Länge um höchstens 15% variieren und die Maschengröße der sich kreuzenden Fäden 1 des Netzschlauches 2 sollte bei entspanntem Netzschlauch, also ohne äußere Krafteinwirkung 20 bis 850000 µm² betragen. Diese Maschengröße gewährleistet eine optimale Abstützung des Ersatzgefäßes.

Zweckmäßig sollte der Netzschlauch über seine gesamte Länge dehnbar oder zusammendrückbar sein, wobei seine elastische Rückstellkraft bei einer Verlängerung auf das Doppelte oder einer Verkürzung auf die Hälfte seiner Länge im entspannten Zustand maximal 30 Pond (0,3 Newton) betragen soll.

Zweckmäßig beträgt der axial gerichtete Winkel zwischen den sich kreuzenden Netzfäden zwischen 70 und 170°. Bei einer bevorzugten Ausführungsform erstrecken sich die sich um die Schlauchachse wendelförmig herumerstreckenden Fäden im spannungslosen Zustand des Schlauches, also vor dessen Stauchen oder Dehnen, überwiegend in einem Winkel von etwa 60° zur Längsrichtung des Schlauches. Dies ermöglicht die größtmögliche gegenseitige Beweglichkeit der Netzfäden im Netzschlauch und gewährleistet, daß der Netzschlauch an beliebigen Stellen seiner Länge bis auf das 10-fache seiner Länge gedehnt oder bis auf ein Fünftel seiner Länge gestaucht werden kann.

Um eine gute Handhabung der Prothese beim Implantieren zu ermöglichen, sollte der Netzschlauch mit dem Blutgefäßabschnitt vorzugsweise durch einen im ausgehärteten Zustand elastischen biologischen Kleber verklebt sein, wodurch ein biologisches Verbundtransplantat erhalten wird, das im Gegensatz zu den bekannten Prothesen knicksicher ist und, wenn ihr Netzschlauch aus Metallfäden besteht, auch im Röntgenverfahren ohne Kontrastmittel abbildbar ist.

Die Erfindung betrifft auch ein Verfahren zur Herstellung einer Gefäßprothese der vorgeschilderten, erfindungsgemäßen Art gemäß Anspruch 15. Dieses Verfahren ist dadurch gekennzeichnet, daß über das aus dem menschlichen oder tierischen Körper entnommene Ersatzgefäß ein sich im wesentlichen an den Außenumfang des Gefäßes anliegender Netzschlauch gezogen wird und auf dem Ersatzgefäß stellenweise in Längsrichtung unter Durchmesserveränderungen gedehnt oder gestaucht und dabei zum ganzflächigen Anliegen an dem Gefäß gebracht wird. Vorzugsweise kann zur Verklebung des Gefäßabschnitts mit dem Netzschlauch Kleber auf den Netzschlauch und durch den Netzschlauch hindurch auf den Gefäßabschnitt, z.B. durch Betupfen und/oder Besprühen, aufgebracht werden und kann der Gefäßabschnitt durch Einbringen eines Innendrucks an den Netzschlauch angepreßt und diese Anpressung über eine Zeit lang, die zur festen Klebeverbindung des Netzschlauches mit dem Gefäßabschnitt notwendig ist, aufrechterhalten wird. Der Kleber kann auch schon vor dem Überschieben des Netzschlauches auf den Gefäßabschnitt aufgebracht werden. Der Innendruck kann durch Einbringen eines gasförmigen oder flüssigen Druckmittels, z.B. einer sterilen Kochsalzlösung, erzeugt werden oder durch Einführen eines aufweitbaren, stabförmigen Ballons in den Gefäßabschnitt von dessen distalem Ende her, der durch Füllung mit einem flüssigen oder gasförmigen Druckmittel, z.B. sterile Kochsalzlösung oder Luft, an den Netzschlauch angepreßt und anschließend nach Entleerung wieder aus dem mit dem Netzschlauch überzogenen Venenabschnitt herausgezogen wird.

Die Erfindung betrifft auch einen Teile-Set gemäß Anspruch 25 zur Herstellung der erfindungsgemäßen Gefäßprothese bzw. zur Durchführung des erfindungsgemäßen Verfahrens. Dieses Set besteht aus einem durch axiales Dehnen oder Stauchen in seinem Durchmesser veränderbaren Netzschlauch mit gegeneinander verschiebbaren, sich kreuzenden Netzfäden, aus einem rohrförmigen Führungsmittel zum Überziehen des Netzschlauches über das Ersatzgefäß und aus einem in das Ersatzgefäß einführbaren Aufweitorgan zum Aufweiten und Anpressen des Ersatzgefäßes an den Netzschlauch besteht.

Vorteilhafte Ausführungsformen der Gefäßprothese, des Verfahrens und des Teile-Sets sind in den Unteransprüchen aufgeführt.

Die erfindungsgemäße Gefäßprothese kann zum Ersatz jeglichen Blutgefäßes in menschlichen oder tierischen Körpern verwendet werden. Besondere Bedeutung mag diese Prothese zur Herstellung von aorta-coronaren Bypässen haben.

In der Zeichnung sind Ausführungsformen der Prothese gemäß der Erfindung sowie Ausführungsformen des Verfahrens zur Herstellung einer solchen Prothese und der dazu notwendigen Hilfsmittel dargestellt, die im folgenden näher beschrieben werden:
- Fig. 1: zeigt eine erste Ausführungsform der erfindungsgemäßen Gefäßprothese in Schrägansicht;
- Fig. 2: zeigt eine Draufsicht auf einen Ausschnitt aus dem Netzschlauch dieser Gefäßprothese in vergrößerter Darstellung;
- Fig. 3: zeigt eine Kreuzung zweier Netzfäden in Schrägansicht in stark vergrößerter Darstellung;
- Fig. 4: ist ein Querschnitt durch die Gefäßprothese nach Linie IV-IV in Fig. 1;
- Fig. 5: zeigt eine zweite Ausführungsform der Gefäßprothese gemäß der Erfindung in Schrägansicht;
- Fig. 6: ist ein Querschnitt durch die Gefäßprothese nach Linie VI-VI in Fig. 5;
- Fig. 7: zeigt eine Ausführungsform des Ballonstabes vor seinem Einführen in einen Abschnitt eines Ersatzblutgefäßes in Schrägansicht;
- Fig. 8: zeigt das Ersatzgefäß mit eingeführtem und befestigten Ballonstab in Schrägansicht;
- Fig. 9: zeigt eine Ausführungsform eines auf ein Führungsrohr aufgezogenen Netzschlauches in Schrägansicht;
- Fig. 10: zeigt den auf das Führungsrohr aufgezogenen Netzschlauch nach seinem Überschieben über das Ersatzblutgefäß mit eingeschobenem Ballonstab in Schrägansicht;
- Fig. 11: veranschaulicht das Entfernen des Ballonstabes aus der fertigen Gefäßprothese;
- Fig. 12: zeigt eine Ausführungsform des Ballonstabes in Schrägansicht in zusammengefaltetem Zustand;
- Fig. 13: zeigt den Ballonstab gemäß Fig. 12 in aufgeblasenem Zustand, in Schrägansicht;
- Fig. 14: zeigt den Netzschlauch in entspanntem Zustand vor dem Aufschieben auf ein Führungsmittel in Schrägansicht;
- Fig. 15: zeigt den Netzschlauch gemäß Fig. 14, aufgeschoben auf ein Führungsrohr, in Schrägansicht;
- Fig. 16: zeigt das Einführen des Ersatzgefäßes in das mit dem Netzschlauch überzogene Führungsrohr;
- Fig. 17: zeigt das Aufweiten des Ersatzblutgefäßes im Netzschlauch nach Entfernen des Führungsrohres, in Schrägansicht;
- Fig. 18: zeigt das Einwickeln des Ersatzblutgefäßes in eine als Führungsmittel dienende Folie in Schrägansicht;
- Fig. 19: veranschaulicht den Netzschlauch, übergezogen über eine zu einem rohrförmigen Führungsmittel gerollte Folie, in Schrägansicht;
- Fig. 20: zeigt eine andere Ausführungsform der als Führungsmittel dienenden, gerollten Folie in Schrägansicht; und
- Fig. 21: zeigt eine weitere Ausführungsform eines Führungsrohres.

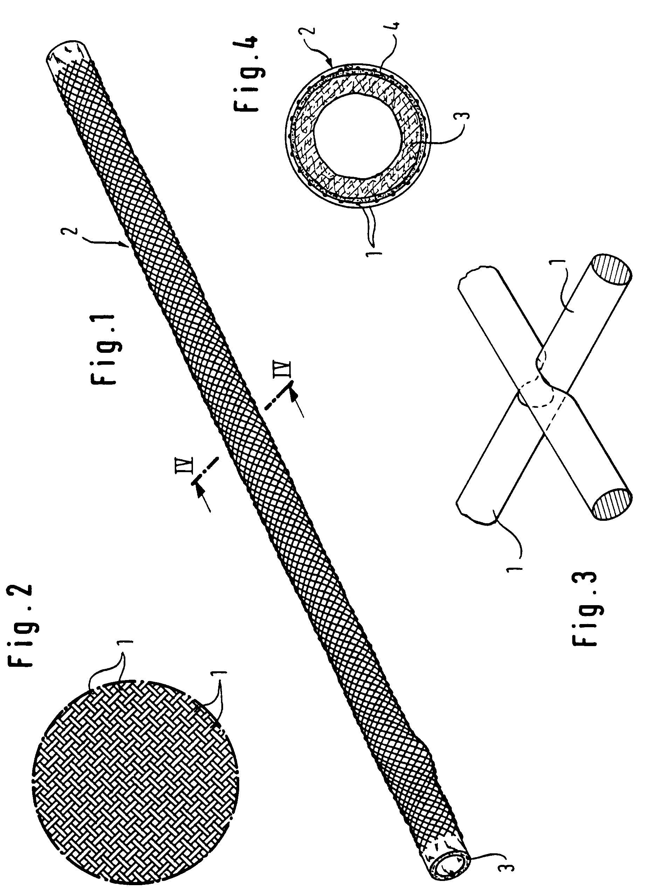

Bei der in Fig. 1 bis 4 dargestellten Ausführungsform der erfindungsgemäßen Gefäßprothese ist ein aus miteinander verflochtenen Fäden 1 gebildeter Netzschlauch 2 über einen aus einem menschlichen oder tierischen Körper entnommenen Abschnitt eines Ersatzblutgefäßes 3 gezogen. An ihren Kreuzungsstellen haben die Netzfäden 1 einen um etwa 50% reduzierten Durchmesser, wie dies in Fig. 3 gezeigt ist. Dies kann durch Elektropolieren im geflochtenen Zustand erreicht werden und hat den Vorteil, daß auch bei Dauerbewegung der Fäden gegeneinander praktisch kein Abrieb erfolgt und die Gefahr des Auftretens von Ermüdungsbrüchen der Fäden stark reduziert ist. Der Netzschlauch ist auf dem Ersatzgefäß 3 stellenweise in Längsrichtung unter Durchmesserveränderung bei gegenseitiger Verschiebung seiner Netzfäden 1 gedehnt oder gestaucht und dadurch zum ganzflächigen, gleichmäßigen Anliegen an den unregelmäßigen Umfang des Ersatzgefäßes gebracht sowie mit dem Ersatzgefäß verklebt. Der Kleber bildet eine Kleberschicht 4, die sich zwischen dem Ersatzgefäß 3 und dem Netzschlauch sowie in den Zwischenräumen zwischen den Fäden 1 des Netzschlauches befindet. Dieser Kleber ist im ausgehärteten Zustand derart elastisch, daß sich die Netzfäden 1 des Netzschlauches 2 gegeneinander verschieben können, so daß der Netzschlauch auch in der fertigen Prothese in einem bestimmten Umfang gestaucht oder gedehnt werden kann, wodurch ein Biegen der Prothese ohne Knickgefahr gewährleistet ist. Neben einer vollflächigen Verklebung ist auch eine nur punktuelle Verklebung zwischen Netzschlauch und Ersatzgefäß möglich.

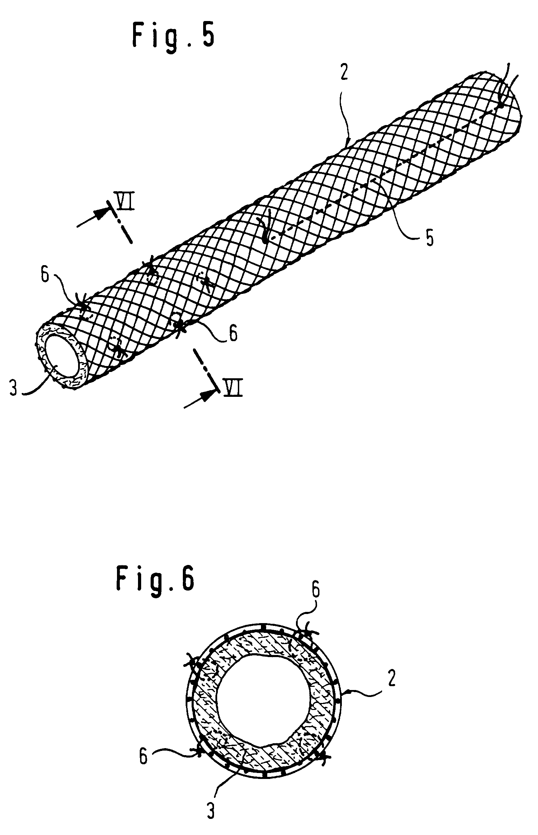

Bei dem in Fig. 5 und 6 dargestellten Ausführungsbeispiel der erfindungsgemäßen Prothese ist der Netzschlauch 2 mit dem Ersatzgefäß 3 nicht durch Verklebung, sondern durch Nähte 5, 6 verbunden. Die Nähte können fortlaufend Nähte 5 oder Einzelknopfnähte 6 sein.

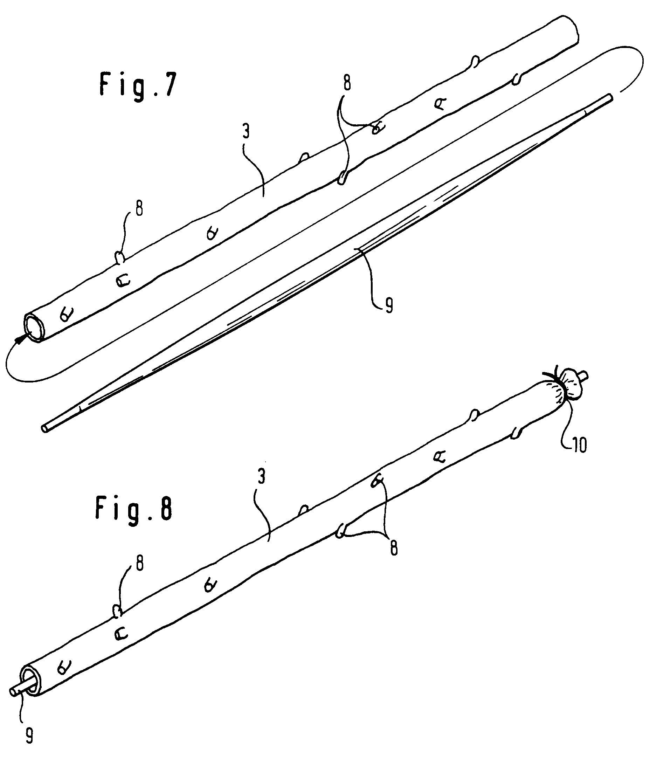

Fig. 7 bis 11 zeigen einzelne Verfahrensschritte bei der Herstellung der in Fig. 1 bis 6 gezeigten Gefäßprothesen, bei der beispielsweise als Ersatzgefäß 3 ein Abschnitt einer aus einem menschlichen oder tierischen Körper entnommenen Vene verwendet wird. Dabei wird zunächst, wie in Fig. 7 und 8 gezeigt, in das Ersatzgefäß 3 z.B. wegen der Venenklappen von dem distalen Gefäßende her ein Ballonstab 9 eingeschoben und an seinem vorderen Ende mit einer Ligatur (Abbindung) 10 am Ersatzgefäß 3 befestigt. Daraufhin wird bei dem hier beschriebenen Ausführungsbeispiel die Oberfläche des Ersatzgefäßes mit einem Kleber versehen, der durch Betupfen und/oder Besprühen aufgetragen werden kann. Die Seitenäste 8 des Ersatzgefäßes können unabgebunden bleiben (Fig. 7 und 8).

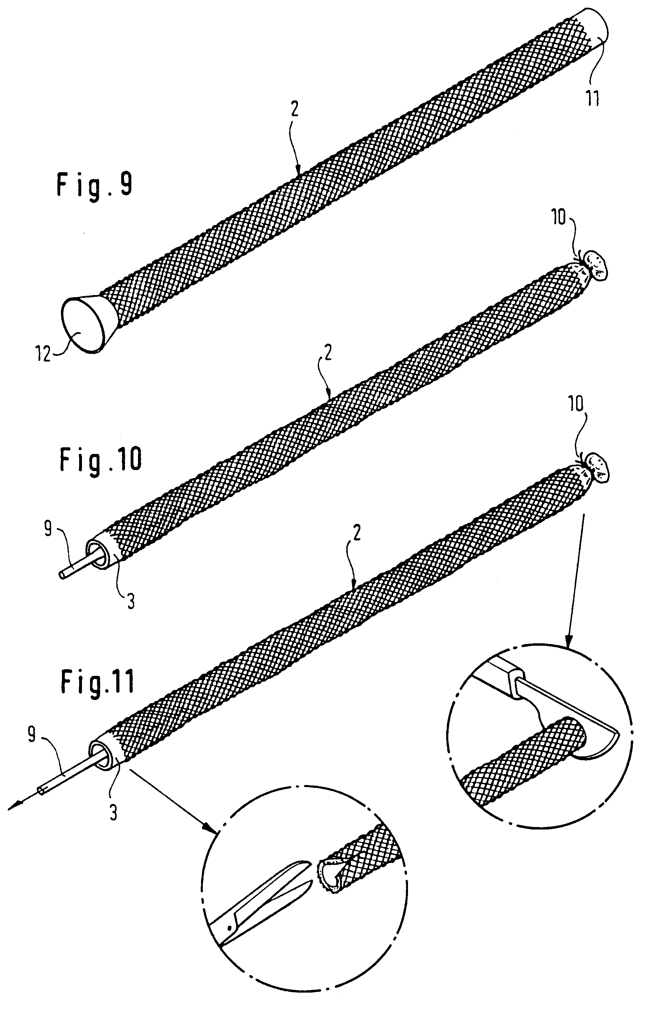

Der auf ein Führungsrohr 11 mit an seinem vorderen Ende angeordneten oder ausgebildeten Führungstrichter 12 aufgezogenen Netzschlauch 2 (Fig. 9) wird anschließend mit Hilfe dieses Führungsrohres auf das vorpräparierte Ersatzgefäß 3 aufgeschoben, und zwar zweckmäßig in Richtung vom proximalen zum distalen Ende des Ersatzgefäßes 3 hin (Fig. 10). Das Führungsrohr hat in diesem Fall etwa gleiche Länge wie das Ersatzgefäß 3. Anschließend wird der Führungstrichter 12 vom vorderen Ende des Führungsrohres 11 abgeschnitten und über das distale Gefäßende hinweg abgezogen, während das Führungsrohr 11 über das proximale Gefäßende hinweg aus dem Netzschlauch 2 herausgezogen und damit von dem Gefäß 3 abgezogen wird. Der das Ersatzgefäß 3 umgebende Netzschlauch 2 kann dann stellenweise in Längsrichtung auf dem Gefäß verschoben und dabei unter Durchmesserveränderung gedehnt oder gestaucht werden. Dadurch kann er mit seinem Durchmesser an den unterschiedlichen Umfang des Ersatzgefäßes angepaßt und zum ganzflächigen Anliegen an das Ersatzgefäß über dessen gesamte Länge hinweg gebracht werden.

Anschließend wird bei dem beschriebenen Verfahrensbeispiel Kleber durch Betupfen oder Besprühen auf die Oberfläche des Netzschlauches 2 und durch dessen Netzmaschen hindurch aufgebracht und durch Aufblasen des Ballonstabes 9 das Ersatzgefäß 3 mit seinem Außenumfang an den Netzschlauch angedrückt. Dies kann durch Anschließen des Ballonstabes an eine Druckluftleitung oder mittels eines in einem Zylinder geführten Kolben, beispielsweise einer Spritze mit Kanüle, erfolgen. Das Andrücken sollte so lange geschehen, wie es für die Erzielung einer festen Klebeverbindung zwischen dem Ersatzgefäß 3 und dem Netzschlauch 2 notwendig ist.

Nach Fertigstellung dieser Verklebung wird der Ballonstab 9 entleert, das mit der Ligatur 10 versehene Ende des Ersatzgefäßes 3 abgeschnitten und der Ballonstab 9 gegebenenfalls nach vorherigem Aufschneiden des anderen Endes des Ersatzgefäßes und übergezogenen Netzschlauches 2 in Pfeilrichtung entfernt (Fig. 11). Die Gefäßprothese liegt nunmehr in der in Fig. 1 gezeigten, fertigen Form vor und kann zur Implantation verwendet werden.

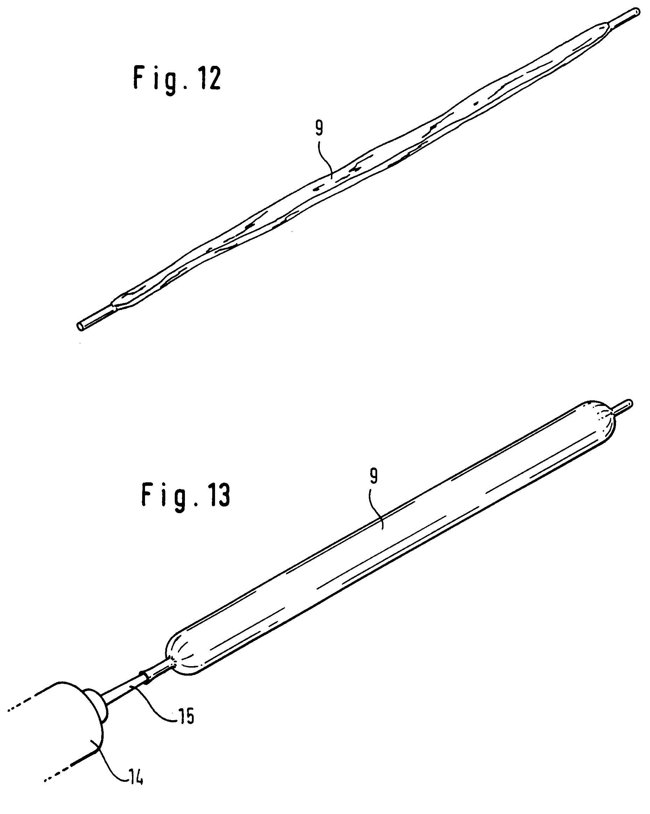

In Fig. 12 bis 21 sind weitere Ausführungsformen der Hilfsmittel zur Herstellung der erfindungsgemäßen Gefäßprothese dargestellt.

Der Ballonstab 9 kann aus dehnbarem Material bestehen und durch Einbringen eines gasförmigen oder flüssigen Druckmittels durch Dehnung aufgeweitet werden. Fig. 12 und 13 zeigen eine Ausführungsform des Ballonstabes 9, bei der der Ballon aus einem im wesentlichen undehnbaren Material besteht. Dieser Ballonstab kann sich im entleerten Zustand zusammenfalten (Fig. 12), während er im mit Druckmittel gefüllten Zustand etwa Wurstform annimmt. Fig. 13 zeigt auch das Einbringen des Druckmittels in den Ballonstab mit Hilfe einer Spritze 14 mit Kanüle 15. Der Ballonstab kann im aufgeblasenen Zustand anstelle einer zylindrischen Form (Wurstform) auch eine konische Form haben, wenn dies aufgrund des verwendeten Ersatzgefäßes zweckmäßig ist.

Wie bereits erwähnt, kann das Aufweiten des Ersatzgefäßes zum Zwecke einer guten Verklebung mit dem übergeschobenen Netzschlauch 2 auch ohne Ballonstab 9 durch direktes Einbringen eines flüssigen oder gasförmigen Druckmittels in das Ersatzgefäß geschehen. Dies kann vom distalen Ende des Ersatzgefäßes her erfolgen, während das proximale Ende abgebunden wird. Zu diesem Zweck kann in das distale Ende eine Kanüle 16 mittels eines Fadens 17 eingebunden sein.

Die Füllung des Ballonstabes kann in allen Fällen unter Anwendung eines Überdruckventils geschehen.

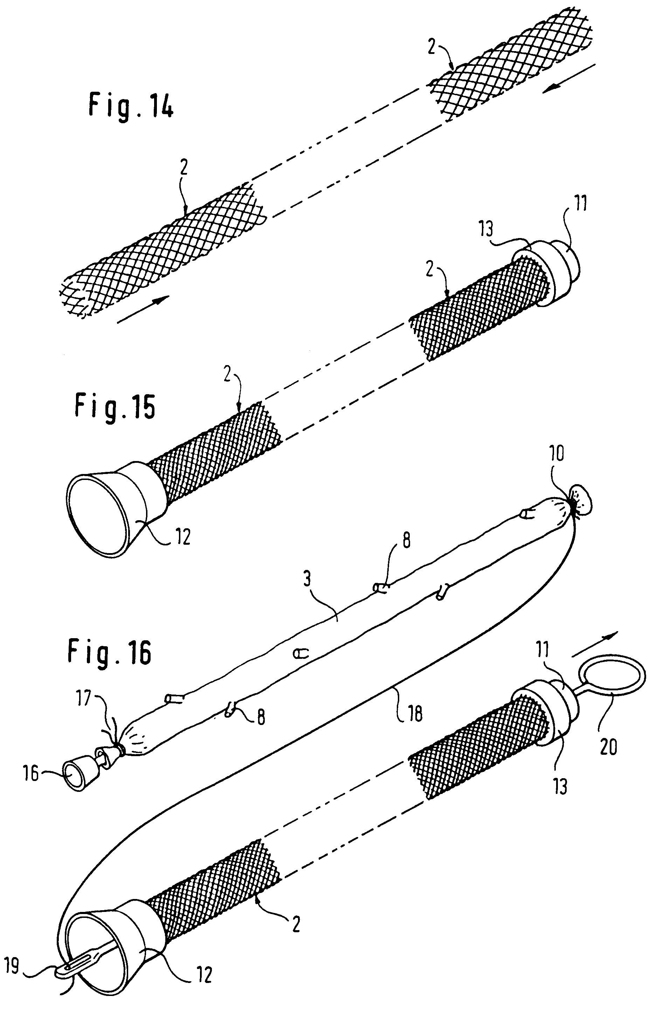

Bei dem in Fig. 15 gezeigten Ausführungsbeispiel hat das zum Überziehen des Netzschlauches 2 über das Ersatzgefäß 3 dehnende Führungsrohr 11 geringere Länge als das Ersatzgefäß im entspannten Zustand (Fig. 14). Dies verringert die Gefahr, daß das aus Präparationsgründen häufig feuchte Ersatzgefäß beim Überschieben des Führungsrohres sich an dessen Wand festsaugt. Da der Netzschlauch 2 im entspannten, also im ungedehnten und ungestauchten Zustand, eine Länge haben soll, die etwa der Länge des Ersatzgefäßes entspricht (Fig. 14), muß der Netzschlauch in diesem Fall vor oder während seines Aufziehens auf das Führungsrohr 11 auf die Länge dies Rohres in Pfeilrichtung (Fig. 14) gestaucht werden. Der gestauchte Zustand des Netzschlauches 2 auf dem Führungsrohr 11 wird durch den Führungstrichter 12 und den Ring 13 aufrechterhalten, die radial über den Umfang des Rohres hinausragen und bei diesem Beispiel auf das Rohr 11 aufsteckbar sind.

Das Einziehen des Ersatzgefäßes 3 in das Führungsrohr 11 mit dem aufgezogenen Netzschlauch 2 kann dadurch erleichtert werden, daß der Faden 18 der Abbindung 10 des proximalen Endes des Erstzgefäßes an das Ende 19 einer Fadenfängerstange 20 angehängt wird, welche vorher in das Führungsrohr 11 eingeschoben worden ist (Fig. 16). Durch Herausziehen der Fadenfängerstange 20 aus dem Führungsrohr kann dann das Ersatzgefäß 3 in das Führungsrohr eingezogen werden. Anschließend kann das Herausziehen des Führungsrohres 11 aus dem Netzschlauch 2 unter Entfernung des Führungstrichters 12 oder des Ringes 13 erfolgen, worauf der zusammengestauchte Netzschlauch 2 sich wieder auf seine ursprüngliche Länge entspannt.

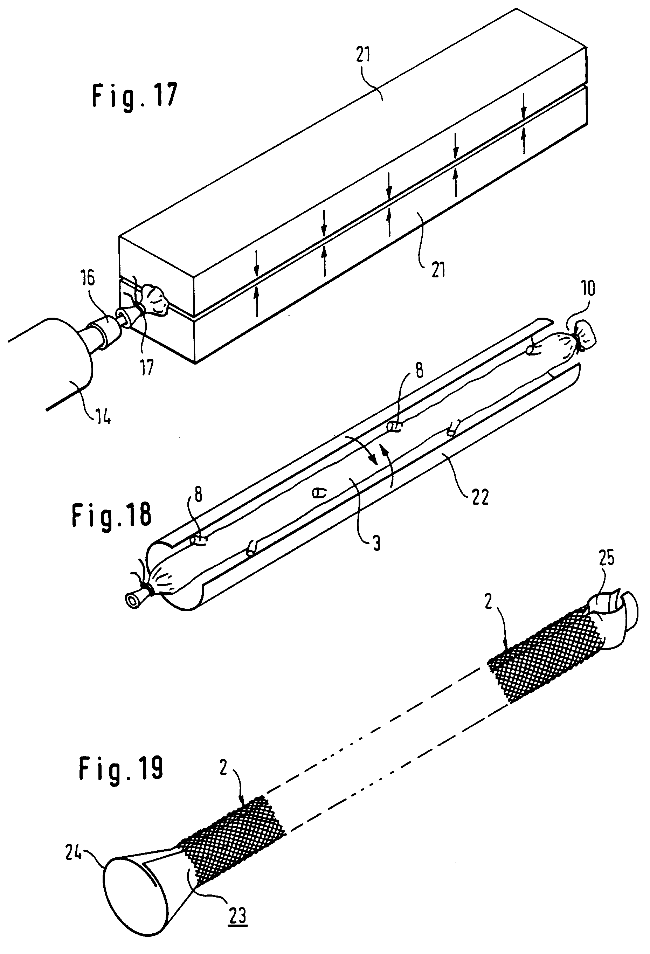

Der Außendurchmesser der Gefäßprothese kann vor dem Verklebungsvorgang durch Anlegen zweiter halbkreisförmiger Schalen 21 aus biokompatiblem Material bestimmt werden, zwischen welche das mit dem Netzschlauch überzogene Ersatzgefäß eingelegt werden kann (Fig. 17).

Das Überziehen des Netzschlauches über das Ersatzgefäß 3 kann auch mit Hilfe einer dünnen Folie 22 erfolgen (Fig. 18). Die Folie kann aus einem biokompatiblen Material, wie Kunststoff oder Metall, bestehen und vorzugsweise eine Dicke von 0,1 bis 0,4 mm haben. Sie besitzt zweckmäßig eine vorgerollte Form, wodurch sie nach dem Einlegen des Ersatzgefäßes in die Folie sich von selbst um das Ersatzgefäß herumwickelt.

In Fig. 19 ist ein Ausführungsbeispiel gezeigt, bei der der Netzschlauch 2 über eine gerollte Folie 23 gezogen ist, die ebenso wie die Folie 22 als Führungsmittel beim Überziehen des Netzschlauches 2 über das Ersatzgefäß 3 dient. Bei diesem Ausführungsbeispiel sind die über die Enden des Netzschlauches 2 hinausragenden Enden 24, 25 der gerollten Folie trichterförmig bzw. kolbenförmig erweitert. Dies kann dazu dienen, den Netzschlauch 2 in einem gestauchten Zustand zu halten, während das trichterförmig aufgeweitete Ende 24 der gerollten Folie 23 auch zum leichteren Überschieben über das Ersatzgefäß 3 dienen kann. Die trichterförmige bzw. kolbenförmige Aufweitung der Enden der Rollfolie kann nach dem Aufziehen des Netzschlauches 2, z.B. durch Thermoverformung, erfolgen.

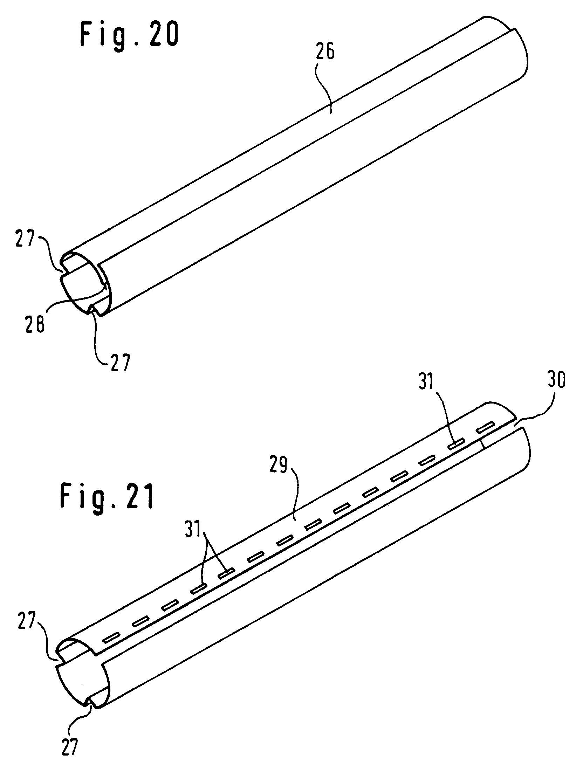

In Fig. 20 ist eine gerollte Folie 26 als rohrförmiges Führungsmittel für das Überziehen des Netzschlauches über das Ersatzgefäß gezeigt, welches mit radial nach innen ragenden Längsrippen 27 versehen ist, die beim Überschieben über das Ersatzgefäß als Längsführung für das Gefäß dienen und damit ein Verdrehen des Gefäßes verhindern. Auch die innenliegende Längskante 28 der gerollten Folie 26 kann dem gleichen Zwecke dienen.

In Fig. 21 ist eine Ausführungsform eines Führungsmittels 29 dargestellt, welches aus einem dünnwandigen Rohr mit einem sich über seine ganze Länge erstreckenden Längsschlitz 30 besteht und mit Perforationen 31 in seiner Wandung ausgestattet ist. Auch dieses aus einem Metall oder Kunststoff bestehende Führungsrohr ist mit radial nach innen ragenden Längsrippen 27 ausgestattet. Die Perforationen 31, die vorzugsweise eine Größe von 0,5 x 5 mm haben, haben den Zweck, das Festsaugen des Ersatzgefäßes an das Führungsrohr bei seinem Überschieben über das Ersatzgefäß zu verhindern. Der Längsschlitz im Führungsrohr kann ebenfalls als Führungs- und Richtmittel für das Ersatzgefäß bei dessen Einziehen in das Rohr dienen, um dessen Verdrehen zu verhindern. Das Führungsrohr kann aber auch ohne Längsschlitz 30 ausgestattet sein.

## Patentansprüche

1. Gefäßprothese für den Ersatz von Blutgefäßen im menschlichen oder tierischen Körper, bestehend aus einem von einem menschlichen oder tierischen Körper entnommenen Abschnitt eines Ersatzblutgefäßes (3) und einem über diesen Gefäßabschnitt gezogenen Netzschlauch (2), dessen den Schlauch bildende, sich kreuzende Fäden (1) sich wendellinienförmig um die Längsachse des Schlauches herumwinden,
dadurch **gekennzeichnet,** daß der Netzschlauch (2) auf dem Ersatzgefäß (3) stellenweise in Längsrichtung unter Durchmesserveränderung gedehnt oder gestaucht und dadurch zum ganzflächigen, gleichmäßigen Anliegen an dem Ersatzgefäß gebracht ist.

2. Prothese nach Anspruch 1, dadurch gekennzeichnet, daß der Durchmesser des Netzschlauches (2) durch dessen axiales Dehnen bis auf das Zehnfache des Durchmessers des entspannten Schlauches verkleinert und durch axiales Stauchen bis auf das Fünffache des Durchmessers des entspannten Schlauches vergrößert werden kann.

3. Prothese nach Anspruch 1, dadurch gekennzeichnet, daß die Fäden (1) des Netzschlauches (2) miteinander verflochten sind.

4. Prothese nach Anspruch 1, dadurch gekennzeichnet, daß die sich kreuzenden Fäden (1) des Netzschlauches (2) gegeneinander verschiebbar sind.

5. Prothese nach Anspruch 1, dadurch gekennzeichnet, daß der Netzschlauch (2) mit dem Blutgefäß (3) durch einen im ausgehärteten Zustand elastischen Kleber verklebt ist, so daß die Netzfäden (1) im Verbund gegeneinander verschiebbar sind.

6. Prothese nach Anspruch 1, dadurch gekennzeichnet, daß das Ersatzgefäß (3) an dem übergezogenen Netzschlauch (2) durch eine oder mehrere Nähte (5, 6) fixiert ist.

7. Prothese nach Anspruch 1, dadurch gekennzeichnet, daß die Netzfäden (1) aus Metall oder einer Metallegierung, insbesondere aus rostfreiem Stahl, bestehen.

8. Prothese nach Anspruch 7, dadurch gekennzeichnet, daß die Netzfäden (1) mit biokompatiblem Material beschichtet sind.

9. Prothese nach Anspruch 1, dadurch gekennzeichnet, daß die Netzfäden (1) aus biokompatiblem Kunststoff bestehen.

10. Prothese nach Anspruch 1, dadurch gekennzeichnet, daß die Netzfäden (1) eine Fadenstärke von 10 bis 200 µm, vorzugsweise 25 bis 50 µm haben.

11. Prothese nach Anspruch 1, dadurch gekennzeichnet, daß der axial gerichtete Winkel zwischen den sich kreuzenden Netzfäden (1) zwischen 70 und 170° beträgt.

12. Prothese nach Anspruch 1, dadurch gekennzeichnet, daß die Größe der von den sich kreuzenden Netzfäden (1) gebildeten Maschen bei entspanntem Netzschlauch 20 bis 850000 µm², vorzugsweise 100 bis 200000 µm² beträgt.

13. Prothese nach Anspruch 1, dadurch gekennzeichnet, daß der Durchmesser der Netzfäden (1) an ihren Kreuzungsstellen um bis zu 50% verringert ist.

14. Prothese nach Anspruch 1, dadurch gekennzeichnet, daß die Rückstellkraft des Netzschlauches (2) bei einer Verlängerung auf das Zweifache oder bei einer Verkürzung auf die Hälfte seiner Länge im entspannten Zustand maximal 30 Pond (0,3 Newton) beträgt.

15. Verfahren zur Herstellung einer Gefäßprothese nach einem der Ansprüche 1 bis 14, dadurch gekennzeichnet, daß über das aus dem menschlichen oder tierischen Körper entnommene Ersatzgefäß (3) ein sich im wesentlichen an den Außenumfang des Gefäßes anlegender Netzschlauch (2) gezogen wird und auf dem Ersatzgefäß stellenweise in Längsrichtung unter Durchmesserveränderungen gedehnt oder gestaucht und dabei zum ganzflächigen Anliegen an dem Gefäß gebracht wird.

16. Verfahren nach Anspruch 15, dadurch gekennzeichnet, daß ein zur Verklebung des Ersatzgefäßes (3) mit dem Netzschlauch (2) dienender Kleber auf den Netzschlauch und oder durch den Netzschlauch hindurch auf den Gefäßabschnitt z.B. durch Betupfen und/oder Besprühen aufgebracht wird und daß dann das Ersatzgefäß durch Anlegen eines Innendrucks an den Netzschlauch angepreßt und diese Anpressung bis zur Kleberaushärtung aufrechterhalten wird.

17. Verfahren nach Anspruch 16, dadurch gekennzeichnet, daß der Innendruck durch Einbringen von Gas oder Flüssigkeit in das Ersatzgefäß erzeugt wird, welches Gas bzw. welche Flüssigkeit nach Beendigung der erwünschten Anpressung wieder aus dem Ersatzgefäß entfernt wird.

18. Verfahren nach Anspruch 16, dadurch gekennzeichnet, daß zum Anlegen des Innendrucks an das Ersatzgefäß (3) in diesen ein aufweitbarer Ballonstab (9) eingeschoben wird, der durch Füllung mit einem flüssigen oder gasförmigen Druckmittel bis zur Kleberaushärtung an den Netzschlauch (2) angepreßt und anschließend nach Entleerung wieder aus dem mit dem Netzschlauch überzogenen Ersatzgefäß herausgezogen wird.

19. Verfahren nach Anspruch 18, dadurch gekennzeichnet, daß das Ersatzgefäß an den in ihn eingeführten Ballonstab (9) z.B. durch eine Ligatur (10) befestigt wird.

20. Verfahren nach Anspruch 15, dadurch gekennzeichnet, daß das Überschieben des Netzschlauches (2) mittels eines rohrförmigen Führungsmittels (11, 22) vorzugsweise aus Metall oder Metallegierung erfolgt, auf welches der Netzschlauch vorher aufgeschoben wird, und daß nach dem Aufschieben des Führungsmittels mit dem Netzschlauch auf das Ersatzgefäß (3) das zwischen diesem Gefäß und dem Netzschlauch befindliche Führungsmittel durch Ziehen entfernt wird.

21. Verfahren nach Anspruch 15, dadurch gekennzeichnet, daß das Ersatzgefäß (3) in eine Folie (22) als Führungsmittel eingewickelt wird und daß die Folie anschließend mit dem Netzschlauch (2) überzogen wird, worauf die Folie vom Ersatzgefäß unter Lagesicherung des Netzschlauches gegenüber dem Ersatzgefäß aus dem Netzschlauch herausgezogen wird.

22. Verfahren nach Anspruch 15, dadurch gekennzeichnet, daß das Ersatzgefäß (3) in ein längsgeschlitztes, elastisch aufweitbares Rohr (21) eingelegt wird, welches anschließend mit dem Netzschlauch (2) überzogen wird, worauf das Rohr unter Lagesicherung des Netzschlauches auf dem Ersatzgefäß aus dem Netzschlauch herausgezogen wird.

23. Verfahren nach Anspruch 15 oder 16, dadurch gekennzeichnet, daß der Netzschlauch (2) mit dem Ersatzgefäß (3) derart verklebt wird, daß die Netzfäden (1) im Verbund gegeneinander verschiebbar sind.

24. Verfahren nach Anspruch 23, dadurch gekennzeichnet, daß als Kleber ein im ausgehärteten Zustand elastischer Kleber, z.B. ein Fibrinkleber, verwendet wird.

25. Set zur Herstellung einer Gefäßprothese nach den Ansprüchen 1 bis 14 bzw. zur Durchführung des Verfahrens nach den Ansprüchen 15 bis 24, dadurch gekennzeichnet, daß es aus einem durch Dehnen oder Stauchen in seiner Längsrichtung in seinem Durchmesser veränderbaren Netzschlauch (2) mit gegeneinander verschiebbaren, sich kreuzenden Netzfäden (1), aus einem rohrförmigen Führungsmittel (11, 22) zum Überziehen des Netzschlauches über das Ersatzgefäß (3) und aus einem in das Ersatzgefäß einführbaren Aufweitorgan (9) zum Aufweiten und Anpressen des Ersatzgefäßes an den Netzschlauch besteht.

26. Set nach Anspruch 25, dadurch gekennzeichnet, daß der Durchmesser des Netzschlauches (2) durch dessen axiales Dehnen bis auf das Zehnfache des Durchmessers des entspannten Schlauches verkleinert und durch axiales Stauchen bis auf das Fünffache des Durchmessers des entspannten Schlauches vergrößert werden kann.

27. Set nach Anspruch 25, dadurch gekennzeichnet, daß die Fäden (1) des Netzschlauches (2) miteinander verflochten sind.

28. Set nach Anspruch 25, dadurch gekennzeichnet, daß die sich kreuzenden Fäden (1) des Netzschlauches (2) gegeneinander verschiebbar sind.

29. Set nach Anspruch 25, dadurch gekennzeichnet, daß die Netzfäden (1) eine Fadenstärke von 10 bis 200 µm, vorzugsweise 25 bis 50 µm haben.

30. Set nach Anspruch 25, dadurch gekennzeichnet, daß der axial gerichtete Winkel zwischen den sich kreuzenden Netzfäden (1) zwischen 70 und 170° beträgt.

31. Set nach Anspruch 25, dadurch gekennzeichnet, daß die Größe der von den sich kreuzenden Netzfäden (1) gebildeten Maschen bei entspanntem Netzschlauch 20 bis 850000 µm², vorzugsweise 100 bis 200000 µm² beträgt.

32. Set nach Anspruch 25, dadurch gekennzeichnet, daß die Rückstellkraft des Netzschlauches (2) bei einer Verlängerung auf das Zweifache oder bei einer Verkürzung auf die Hälfte seiner Länge im entspannten Zustand maximal 30 Pond (0,3 Newton) beträgt.

33. Set nach Anspruch 25, dadurch gekennzeichnet, daß das rohrförmige Führungsmittel (22) aus einer Folie besteht, in welches das Ersatzgefäß (3) eingewickelt werden kann.

34. Set nach Anspruch 25, dadurch gekennzeichnet, daß das rohrformige Führungsmittel (11) aus einem längsgeschlitzten, elastisch aufweitbaren Rohr besteht, welches vom Netzschlauch (2) überzogen werden und mit diesem auf das Ersatzgefäß (3) aufgeschoben werden kann.

35. Set nach Anspruch 25, dadurch gekennzeichnet, daß das rohrförmige Führungsmittel (11, 22) an mindestens einem Ende einen vergrößerten Außendurchmesser hat.

36. Set nach Anspruch 25, dadurch gekennzeichnet daß, das rohrförmige Führungsmittel (11, 22) an mindestens einem Ende trichter- oder ringförmig aufgeweitet ist.

37. Set nach Anspruch 29, dadurch gekennzeichnet, daß das trichterförmig oder ringförmig aufgeweitete Ende des rohrförmigen Führungsmittels von einem abziehbaren Trichter (12) oder Ring (13) gebildet ist.

38. Set nach Anspruch 25, dadurch gekennzeichnet, daß das rohrförmige Führungsmittel (11, 22) eine in dessen Innenraum hineinragende Längskante (27) hat, die sich über mindestens einen Großteil der Längsausdehnung des Führungsmittels erstreckt.

39. Set nach Anspruch 38, dadurch gekennzeichnet, daß die Längskante (27) von einer Abknickung oder Einwölbung der Wandung des rohrförmigen Führungsmittels gebildet ist.

40. Set nach Anspruch 25, dadurch gekennzeichnet, daß die Wandung des rohrförmigen Führungsmittels (11, 22) mit Perforationen (31) versehen ist.

41. Set nach Anspruch 25, dadurch gekennzeichnet, daß das Aufweitorgan von einem aufweitbaren Ballonstab (9) gebildet ist, der in das Ersatzgefäß (3) einschiebbar ist.

42. Set nach Anspruch 41, dadurch gekennzeichnet, daß der Ballonstab (9) ein zusammenfaltbarer Schlauch ist, der durch Füllen mit einem Druckmittel auseinanderfaltbar ist.

43. Set nach Anspruch 42, dadurch gekennzeichnet, daß der Ballonstab (9) ein im Querschnitt dehnbarer Schlauch ist, der vor Aufweitung in das Ersatzgefäß (3) einschiebbar ist.

44. Set nach Anspruch 25, dadurch gekennzeichnet, daß es zwei oder mehr teilkreisförmige Schalen (21) aufweist, in welche das mit dem Netzschlauch (2) überzogene Ersatzgefäß (3) einlegbar ist und die dazu bestimmt sind, den Netzschlauch beim Aufweiten und Anpressen des Ersatzgefäßes an seinem Außenumfang abzustützen.

## Claims

1. Prosthetic vessel for replacing blood vessels in the human or animal body, comprising a portion of a replacement blood vessel (3) removed from a human or animal body and a tube net (2) drawn over this vessel portion, and whose interlacing threads (1) forming the tube wind around the longitudinal axis of the tube in a spiral configuration,
characterised in that the tube net (2) is locally stretched or pushed together on the replacement vessel (3), altering its diameter, and thus is brought into whole-surface uniform contact with the replacement vessel.

2. Prosthesis according to claim 1, characterised in that the diameter of the tube net (2) can be reduced by being axially stretched up to ten times the diameter of the relaxed tube, and can be enlarged by being axially pushed together up to five times the diameter of the relaxed tube.

3. Prosthesis according to claim 1, characterised in that the threads (1) of the tube net (2) are braided together.

4. Prosthesis according to claim 1, characterised in that the intersecting threads (1) of the tube net (2) are movable relative to one another.

5. Prosthesis according to claim 1, characterised in that the tube net (2) is glued to the blood vessel (3) by an adhesive which is elastic in the hardened condition, so that the net threads are movable relative to one another in the bond.

6. Prosthesis according to claim 1, characterised in that the replacement vessel (3) is fixed on the tube net (2) drawn over it by one or a plurality of seams (5, 6).

7. Prosthesis according to claim 1, characterised in that the net threads (1) consist of a metal or a metal alloy, particularly of stainless steel.

8. Prosthesis according to claim 7, characterised in that the net threads (1) are coated with biocompatible material.

9. Prosthesis according to claim 1, characterised in that the net threads (1) consist of biocompatible plastics.

10. Prosthesis according to claim 1, characterised in that the net threads (1) have a thread thickness of 10 to 200 µm, preferably 25 to 50 µm.

11. Prosthesis according to claim 1, characterised in that the axial-oriented angle between the intersecting net threads (1) comes to between 70 and 170°.

12. Prosthesis according to claim 1, characterised in that the size of the meshes formed by the intersecting net threads (1) in the relaxed tube net comes to 20 to 850000 µm², preferably 100 to 200000 µm².

13. Prosthesis according to claim 1, characterised in that the diameter of the net threads (1) is reduced by up to 50% at their points of intersection.

14. Prosthesis according to claim 1, characterised in that the return force of the tube net (2) upon elongation to twice or shortening to half of its length in the relaxed state comes to a maximum 30 Pond (0.3 Newton).

15. Method of manufacturing a prosthetic vessel according to one of claims 1 to 14, characterised in that there is drawn over the replacement vessel (3), removed from the human or animal body, a tube net (2) substantially applied to the outer circumference of the vessel, said tube net (2) being locally stretched or pushed together on the replacement vessel in the longitudinal direction, causing changes in diameter, thus being brought into whole-surface contact with the vessel.

16. Method according to claim 15, characterised in that an adhesive serving to glue the replacement vessel (3) to the tube net (2) is applied to the tube net and/or through the tube net on to the vessel portion e.g. by dabbing and/or spraying, and in that the replacement vessel is then pressed against the tube net by application of an internal pressure, and this pressure is maintained until the adhesive hardens.

17. Method according to claim 16, characterised in that the internal pressure is generated by the introduction of gas or liquid into the replacement vessel, said gas or liquid being removed again from the replacement vessel after termination of the desired contact pressure.

18. Method according to claim 16, characterised in that, in order to apply the internal pressure to the replacement vessel (3), an expandable rod balloon (9) is introduced therein, said rod balloon being pressed against the tube net (2) by means of filling with a liquid or gaseous pressure medium until the adhesive hardens, said rod balloon then being withdrawn after emptying out of the replacement vessel covered by the tube net.

19. Method according to claim 18, characterised in that the replacement vessel is secured to the rod balloon (9) introduced therein e.g. by a ligature (10).

20. Method according to claim 15, characterised in that the tube net (2) is pushed over by means of a tubular guide means (11, 22) preferably made of metal or a metal alloy, upon which the tube net is previously pushed, and in that, after the guide means with the net tube has been pushed on to the replacement vessel (3), the guide means located between this vessel and the tube net is withdrawn by pulling.

21. Method according to claim 15, characterised in that the replacement vessel (3) is wound into a film (22) as a guide means, and in that the film is then covered by the tube net (2), whereupon the film is withdrawn from the replacement vessel out of the tube net, the position of the tube net relative to the replacement vessel being secured.

22. Method according to claim 15, characterised in that the replacement vessel (3) is inserted into a longitudinally slotted elastically expandable tube (21), which is then covered by the tube net (2), whereupon the tube is withdrawn from the tube net, the position of the tube net on the replacement vessel being secured.

23. Method according to claim 15 or 16, characterised in that the tube net (2) is glued to the replacement vessel (3) in such a way that the net threads (1) in the bond are movable relative to one another.

24. Method according to claim 23, characterised in that an adhesive which is elastic in the hardened condition, e.g. a fibrin adhesive, is used.

25. Set for manufacturing a prosthetic vessel according to claims 1 to 14 or for carrying out the method according to claims 15 to 24, characterised in that it consists of a tube net (2) alterable in its diameter by stretching or by being pushed together in its longitudinal direction, with intersecting net threads (1) movable relative to one another, of a tubular guide means (11, 22) for drawing the tube net over the replacement vessel (3), and of an expanding member (9) introducible into the replacement vessel for expanding and pressing the replacement vessel against the tube net.

26. Set according to claim 25, characterised in that the diameter of the tube net (2) can be reduced by being axially stretched up to ten times the diameter of the relaxed tube and can be enlarged by being pushed together axially up to five times the diameter of the relaxed tube.

27. Set according to claim 25, characterised in that the threads (1) of the tube net (2) are braided together.

28. Set according to claim 25, characterised in that the intersecting threads (1) of the tube net (2) are movable relative to one another.

29. Set according to claim 25, characterised in that the net threads (1) have a thread thickness of 10 to 200 µm, preferably 25 to 50 µm.

30. Set according to claim 25, characterised in that the axially-oriented angle between the intersecting net threads (1) comes to between 70 and 170°.

31. Set according to claim 25, characterised in that the size of the meshes formed by the intersecting net threads (1) in the relaxed tube net comes to 20 to 850000 µm², preferably 100 to 200000 µm².

32. Set according to claim 25, characterised in that the return force of the tube net (2) during an elongation to twice or during shortening to half of its length in the relaxed condition comes to a maximum 30 Pond (0.3 Newton).

33. Set according to claim 25, characterised in that the tubular guide means (22) comprises a film in which the replacement vessel (3) can be wound.

34. Set according to claim 25, characterised in that the tubular guide means (11) comprises a longitudinally slotted, elastically expandable tube, which can have the tube net (2) drawn over it, and which can, with the latter, be pushed over the replacement vessel (3).

35. Set according to claim 25, characterised in that the tubular guide means (11, 22) has an enlarged outer diameter at at least one end.

36. Set according to claim 25, characterised in that the tubular guide means (11, 22) is expanded in a funnel or annular shape at at least one end.

37. Set according to claim 29, characterised in that the end of the tubular guide means expanded in a funnel or annular shape is formed by a removable funnel (12) or ring (13).

38. Set according to claim 25, characterised in that the end of the tubular guide means (11, 22) has a longitudinal edge (27) projecting into its inner cavity, and which extends over at least a large proportion of the longitudinal extent of the guide means.

39. Set according to claim 38, characterised in that the longitudinal edge (27) is formed by a kink or curvature of the wall of the tubular guide means.

40. Set according to claim 25, characterised in that the wall of the tubular guide means (11, 22) is provided with perforations (31).

41. Set according to claim 25, characterised in that the expanding member is formed by an expandable rod balloon (9) which is introducible into the replacement vessel (3).

42. Set according to claim 41, characterised in that the rod balloon (9) is a foldable tube, which may be unfolded by being filled with a pressure medium.

43. Set according to claim 42, characterised in that the rod balloon (9) is a tube with an expandable cross-section, which is introducible into the replacement vessel (3) before being expanded.

44. Set according to claim 25, characterised in that it has two or a plurality of partially circular shells (21) into which the replacement vessel (3) with the tube net (2) drawn over may be inserted, and which is intended to support the tube net during expansion and pressure of the replacement vessel against its outer circumference.

## Revendications

1. Prothèse vasculaire destinée au remplacement de vaisseaux sanguins dans le corps humain ou animal, constitué d'un tronçon de vaisseau sanguin de substitution (3) prélevé du corps humain ou animal, et d'un boyau de filet (2) recouvrant ce tronçon de vaisseau, et dont les fils (1) qui se croisent et forment le boyau, s'enroulent en hélice autour de l'axe longitudinal du boyau, **caractérisée** en ce que le boyau de filet (2) est, par endroits sur le vaisseau de substitution (3), allongé ou comprimé en direction longitudinale, en produisant une variation de diamètre, et est ainsi appliqué de manière uniforme sur toute la surface contre le vaisseau de substitution.

2. Prothèse selon la revendication 1, **caractérisée** en ce que le diamètre du boyau de filet (2), par son allongement axial, peut être réduit jusqu'à dix fois le diamètre du boyau détendu, et par compression axiale, il peut être agrandi jusqu'à cinq fois le diamètre du boyau détendu.

3. Prothèse selon la revendication 1, **caractérisée** en ce que les fils (1) du boyau de filet (2) sont entrelacés.

4. Prothèse selon la revendication 1, **caractérisée** en ce que les fils (1) du boyau de filet (2), qui se croisent, peuvent coulisser les uns par rapport aux autres.

5. Prothèse selon la revendication 1, **caractérisée** en ce que le boyau de filet (2) est lié par collage au vaisseau sanguin (3) par une colle élastique à l'état durci, de sorte que les fils (1) du filet peuvent coulisser les uns par rapport aux autres dans l'ensemble composite.

6. Prothèse selon la revendication 1, **caractérisée** en ce que le vaisseau de substitution (3) est fixé au boyau de filet (2) de revêtement, par une ou plusieurs sutures (5, 6).

7. Prothèse selon la revendication 1, **caractérisée** en ce que les fils (1) du filet sont réalisés en métal ou en un alliage métallique, notamment en acier inoxydable.

8. Prothèse selon la revendication 7, **caractérisée** en ce que les fils (1) du filet sont revêtus d'un matériau biocompatible.

9. Prothèse selon la revendication 1, **caractérisée** en ce que les fils (1) du filet sont réalisés en une matière plastique biocompatible.

10. Prothèse selon la revendication 1, **caractérisée** en ce que les fils (1) du filet présentent une épaisseur de fil de 10 à 200 µm, de préférence de 25 à 50 µm.

11. Prothèse selon la revendication 1, **caractérisée** en ce que l'angle orienté axialement, entre les fils (1) du filet, qui se croisent, se situe entre 70 et 170°.

12. Prothèse selon la revendication 1, **caractérisée** en ce que la grandeur des mailles formées par les fils (1) du filet, qui se croisent, a une valeur, à l'état détendu du boyau de filet, de 20 à 850000 µm², de préférence de 100 à 200000 µm².

13. Prothèse selon la revendication 1, **caractérisée** en ce que le diamètre des fils (1) du filet est réduit jusqu'à 50% aux endroits des croisements.

14. Prothèse selon la revendication 1, **caractérisée** en ce que la force de rappel du boyau de filet (2), pour un allongement au double de sa longueur ou un raccourcissement de la moitié de sa longueur à l'état détendu, vaut au maximum 30 Pond (0,3 Newton).

15. Procédé de fabrication d'une prothèse vasculaire selon l'une des revendications 1 à 14, **caractérisé** en ce que l'on fait passer par-dessus le vaisseau de substitution (3) prélevé du corps humain ou animal, un boyau de filet (2) qui s'applique sensiblement sur la périphérie extérieure du vaisseau, et que l'on allonge ou comprime dans la direction longitudinale, par endroits sur le vaisseau de substitution, en produisant ainsi son application, sur toute la surface, contre le vaisseau de substitution.

16. Procédé selon la revendication 15, **caractérisé** en ce qu'une colle servant à réaliser une liaison par collage entre le vaisseau de substitution (3) et le boyau de filet (2) est appliquée sur le boyau de filet et/ou au travers du boyau de filet sur le tronçon de vaisseau, par exemple par tamponnage et/ou pulvérisation, et en ce que le vaisseau de substitution est ensuite pressé contre le boyau de filet, par l'application d'une pression intérieure, ce pressage étant maintenu jusqu'à durcissement de la colle.

17. Procédé selon la revendication 16, **caractérisé** en ce que la pression intérieure est engendrée en introduisant du gaz ou du liquide dans le vaisseau de substitution, ce gaz ou ce liquide étant éliminé après achèvement du pressage souhaité.

18. Procédé selon la revendication 16, **caractérisé** en ce que pour appliquer la pression intérieure au vaisseau de substitution (3), on introduit dans celui-ci un ballon tubulaire (9) expansible, qui, par remplissage avec un agent de pression liquide ou gazeux est pressé contre le boyau de filet (2) jusqu'à durcissement de la colle, et après avoir été vidé, est à nouveau extrait du vaisseau de substitution revêtu du boyau de filet.

19. Procédé selon la revendication 18, **caractérisé** en ce que le vaisseau de substitution est fixé au ballon tubulaire (9) qui est introduit dans ce vaisseau, par exemple au moyen d'une ligature (10)

20. Procédé selon la revendication 15, **caractérisé** en ce que la mise en place du boyau de filet (2) s'effectue à l'aide d'un moyen de guidage (11, 22) de forme tubulaire, de préférence en métal ou alliage métallique, sur lequel est emmanché auparavant le boyau de filet, et en ce qu'après la mise en place du moyen de guidage avec le boyau de filet sur le vaisseau de substitution (3), on retire, par traction, le moyen de guidage se trouvant entre ce vaisseau et le boyau de filet.

21. Procédé selon la revendication 15, **caractérisé** en ce que le vaisseau de substitution (3) est enroulé dans une feuille (22) en tant que moyen de guidage, et en ce que la feuille est ensuite revêtue par le boyau de filet (2), la feuille étant ensuite extraite du boyau de filet, par traction, tout en maintenant la position du boyau de filet par rapport au vaisseau de substitution.

22. Procédé selon la revendication 15, **caractérisé** en ce que le vaisseau de substitution (3) est déposé dans un tube (21) fendu longitudinalement et expansible de manière élastique, qui est ensuite revêtu par le boyau de filet (2), le tube étant ensuite extrait du boyau de filet, par traction, tout en maintenant la position du boyau de filet sur le vaisseau de substitution.

23. Procédé selon la revendication 15 ou 16, **caractérisé** en ce que le boyau de filet (2) est relié par collage au vaisseau de substitution (3) de manière telle, que le fils (1) du filet peuvent coulisser les uns par rapport aux autres dans l'ensemble composite.

24. Procédé selon la revendication 23, **caractérisé** en ce que l'on utilise en guise de colle, une colle élastique à l'état durci, par exemple une colle à la fibrine.

25. Set de fabrication d'une prothèse vasculaire selon les revendications 1 à 14, et destiné à mettre en oeuvre le procédé selon les revendications 15 à 24, **caractérisé** en ce qu'il est constitué d'un boyau de filet (2) dont le diamètre peut être modifié par allongement et ou compression dans sa direction longitudinale, et comportant des fils (1) de filet qui se croisent et peuvent coulisser les uns par rapport aux autres, d'un moyen de guidage (11, 22) de forme tubulaire, destiné mettre en place le boyau de filet par-dessus le vaisseau de substitution (3), et d'un organe d'expansion (9) pouvant être introduit dans le vaisseau de substitution et destiné à évaser et à presser le vaisseau de substitution contre le boyau de filet.

26. Set selon la revendication 25, **caractérisé** en ce que le diamètre du boyau de filet (2) peut, par son allongement axial, être réduit jusqu'à dix fois le diamètre du boyau à l'état détendu, et, par compression axiale, être agrandi jusqu'à cinq fois le diamètre du boyau à l'état détendu.

27. Set selon la revendication 25, **caractérisé** en ce que les fils (1) du boyau de filet (2) sont entrelacés.

28. Set selon la revendication 25, **caractérisé** en ce que les fils (1) du boyau de filet (2), qui se croisent, peuvent coulisser les uns par rapport aux autres.

29. Set selon la revendication 25, **caractérisé** en ce que les fils (1) du filet présentent une épaisseur de fil de 10 à 200 µm, de préférence de 25 à 50 µm.

30. Set selon la revendication 25, **caractérisé** en ce que l'angle orienté axialement, entre les fils (1) du filet, qui se croisent, se situe entre 70 et 170°.

31. Set selon la revendication 25, **caractérisé** en ce que la grandeur des mailles formées par les fils (1) du filet, qui se croisent, a une valeur, à l'état détendu du boyau de filet, de 20 à 850000 µm², de préférence de 100 à 200000 µm².

32. Set selon la revendication 25, **caractérisé** en ce que la force de rappel du boyau de filet (2), pour un allongement au double de sa longueur ou un raccourcissement de la moitié de sa longueur à l'état détendu, vaut au maximum 30 Pond (0,3 Newton).

33. Set selon la revendication 25, **caractérisé** en ce que le moyen de guidage (22) de forme tubulaire est constitué d'une feuille dans laquelle peut être enroulé le vaisseau de substitution (3).

34. Set selon la revendication 25, **caractérisé** en ce que le moyen de guidage de forme tubulaire (11) est formé par un tube fendu longitudinalement et expansible de manière élastique, qui peut être revêtu par le boyau de filet (2) et peut être emmanché avec celui-ci, par-dessus le vaisseau de substitution (3).

35. Set selon la revendication 25, **caractérisé** en ce que le moyen de guidage (11, 22) de forme tubulaire présente, à au moins une extrémité, un diamètre extérieur agrandi.

36. Set selon la revendication 25, **caractérisé** en ce que le moyen de guidage (11, 22) de forme tubulaire est évasé en forme d'entonnoir ou de collerette annulaire, à au moins une extrémité.

37. Set selon la revendication 29, **caractérisé** en ce que l'extrémité évasée en forme d'entonnoir ou de collerette annulaire du moyen de guidage de forme tubulaire, est formée par un entonnoir (12) ou une collerette annulaire (13).

38. Set selon la revendication 25, **caractérisé** en ce que le moyen de guidage (11, 22) de forme tubulaire présente une arête longitudinale (27) pénétrant dans son espace intérieur et qui s'étend au moins sur une plus grande partie de l'étendue longitudinale du moyen de guidage.

39. Set selon la revendication 38, **caractérisé** en ce que l'arête longitudinale (27) est formée par un repli ou une empreinte de la paroi du moyen de guidage de forme tubulaire.

40. Set selon la revendication 25, **caractérisé** en ce que la paroi du moyen de guidage (11, 22) de forme tubulaire est pourvue de perforations (31).

41. Set selon la revendication 25, **caractérisé** en ce que l'organe d'expansion est formé par un ballon tubulaire (9) qui peut être inséré dans le vaisseau de substitution (3).

42. Set selon la revendication 41, **caractérisé** en ce que le ballon tubulaire (9) est un boyau repliable, qui peut se déplier par remplissage avec un agent de pression.

43. Set selon la revendication 42, **caractérisé** en ce que le ballon de forme tubulaire (9) est un boyau pouvant être dilaté en section transversale, qui peut être inséré dans le vaisseau de substitution (3) avant l'expansion.

44. Set selon la revendication 25, **caractérisé** en ce qu'il comporte deux coques (21) ou plus de forme circulaire partielle, dans lesquelles peut être déposé le vaisseau de substitution (3) revêtu du boyau de filet (2), et qui sont destinées à soutenir le boyau de filet sur sa périphérie extérieure, lors de l'expansion et de l'application par pressage du vaisseau de substitution.
